(19) **Europäisches Patentamt / European Patent Office / Office européen des brevets**

(11) **EP 4 042 194 B1**

(12) **FASCICULE DE BREVET EUROPEEN**

(45) Date de publication et mention de la délivrance du brevet:
**08.11.2023 Bulletin 2023/45**

(21) Numéro de dépôt: **20785521.4**

(22) Date de dépôt: **07.10.2020**

(51) Classification Internationale des Brevets (IPC):
**G01S 13/00** (2006.01)

(52) Classification Coopérative des Brevets (CPC):
**G01S 13/88; A61B 5/1117; A61B 5/7264; G01S 7/417**

(86) Numéro de dépôt international:
**PCT/EP2020/078161**

(87) Numéro de publication internationale:
**WO 2021/069518 (15.04.2021 Gazette 2021/15)**

(54) **DISPOSITIF DE CARACTÉRISATION DE L'ACTIMÉTRIE D'UN SUJET EN TEMPS RÉEL**

VORRICHTUNG ZUR CHARAKTERISIERUNG DER AKTIMETRIE EINES SUBJEKTS IN ECHTZEIT

DEVICE FOR CHARACTERISING THE ACTIMETRY OF A SUBJECT IN REAL TIME

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **07.10.2019 EP 19306308**

(43) Date de publication de la demande:
**17.08.2022 Bulletin 2022/33**

(73) Titulaires:
• **Centre national de la recherche scientifique
75016 Paris (FR)**
• **École Nationale Supérieure de l'Électronique et de
ses Applications
95800 Cergy (FR)**
• **The University Court of the University
of Glasgow
Glasgow G12 8QQ (GB)**
• **CY Cergy Paris Université
95000 Cergy (FR)**

(72) Inventeurs:
• **ROMAIN, Olivier
91230 MONTGERON (FR)**
• **LEKERNEC, Julien
29300 QUIMPERLE (FR)**
• **LORANDEL, Jordane
78700 CONFLANS SAINTE HONORINE (FR)**
• **FIORANELLI, Francesco
2628 RG DELFT Zuid Holland (NL)**

(74) Mandataire: **A.P.I. Conseil
Immeuble Newton
4, rue Jules Ferry
64000 Pau (FR)**

(56) Documents cités:
• **HE MI ET AL: "Human Fall Detection Based on Machine Learning Using a THz Radar System", 2019 IEEE RADAR CONFERENCE (RADARCONF), IEEE, 22 avril 2019 (2019-04-22), pages 1-5, XP033616856, DOI: 10.1109/RADAR.2019.8835828 [extrait le 2019-09-12]**
• **WU QISONG ET AL: "Radar-based fall detection based on Doppler time-frequency signatures for assisted li", IET RADAR SONAR NAVIGATION, THE INSTITUTION OF ENGINEERING AND TECHNOLOGY, UK, vol. 9, no. 2, 1 février 2015 (2015-02-01), pages 164-172, XP006051698, ISSN: 1751-8784, DOI: 10.1049/IET-RSN.2014.0250**
• **JAVIER RIOS JESUS ET AL: "Application of Linear Predictive Coding for Human Activity Classification Based on Micro-Doppler Signatures", IEEE GEOSCIENCE AND REMOTE SENSING LETTERS, IEEE SERVICE CENTER, NEW YORK, NY, US, vol. 11, no. 10, 1 octobre 2014 (2014-10-01), pages 1831-1834, XP011548152, ISSN: 1545-598X, DOI: 10.1109/LGRS.2014.2311819 [extrait le 2014-05-12]**

- FIORANELLI FRANCESCO ET AL: "Bistatic human micro-Doppler signatures for classification of indoor activities", 2017 IEEE RADAR CONFERENCE (RADARCONF), IEEE, 8 mai 2017 (2017-05-08), pages 610-615, XP033104089, DOI: 10.1109/RADAR.2017.7944276 [extrait le 2017-06-07]

**Description**

**DOMAINE DE L'INVENTION**

La présente invention concerne un dispositif pour caractériser en temps réel l'actimétrie d'un sujet.

**ETAT DE LA TECHNIQUE**

La détection automatique de posture a donné lieu ces dernières années à une intense activité de recherche et des grandes retombées économiques. Les capteurs 3D type en anglais « Kinect » en particulier ont permis de changer de paradigme dans le jeu en offrant la possibilité de prendre en compte l'information de profondeur (3D) et ainsi de discriminer de manière plus efficace les différents types de mouvements des sujets.
On retrouve aujourd'hui la plupart de ces systèmes de mesure et d'analyse des mouvements tant dans le milieu de l'animation virtuelle (simulation de la marche) que dans le domaine biomécanique et médical.
Ces systèmes permettent d'étudier des problématiques de détection de pathologie du sujet, d'analyser le corps en activité, ou de comprendre les mécanismes de la marche.
Les compétitions académiques encore récemment organisés sur cette thématique montrent néanmoins les limitations des capteurs actuels, notamment pour la reconnaissance de posture dans diverses situations et en particulier les chutes de sujets déficients.
Les systèmes actuels peuvent être regroupés en trois catégories ; les systèmes embarqués sur un sujet, les systèmes déportés et les systèmes hybrides (mixte d'embarqués et de déportés).
De nombreuses technologies embarquées ont été proposées pour la surveillance des sujets et spécifiquement pour la détection des chutes [3], ces trente dernières années. Celles-ci incluent les dispositifs portables tels que les podomètres, locomètres, accéléromètres, les gyroscopes et les boutons-poussoirs de panique, les capteurs inertiels tels que les smartphones, les capteurs infrarouges, vibratoires, acoustiques, magnétiques,
**[0008]** Bien que ces dispositifs donnent de bons résultats [4] sur l'identification de chutes (98%), la plupart de ces solutions embarquées sur le sujet souffrent de plusieurs problèmes majeurs [2] qui limitent leurs usages :

- Ils doivent être portés, ce qui dépend de la conformité de l'utilisateur ou d'y penser si vous vous réveillez la nuit pour aller aux toilettes.
- Ils sont facilement cassables en cas de chute, de choc ou si on s'assoie dessus.
- Ils doivent être rechargés, ce qui peut être difficile pour les sujets ayant une déficience cognitive.
- Le taux de fausses alarmes.
- Ils sont stigmatisant pour les sujets.
- Ils ne respectent pas (pour certains) la vie privée.

Les dispositifs déportés sont généralement basés sur l'utilisation de systèmes de mesure intégrés dans le lieu de vie de l'usager. On retrouve l'emploi de caméra(s) vidéo [5], les tapis roulants de marche, les capteurs photographiques RGB-D pour « Red, Green, Blue detector » selon une terminologie anglo-saxonne et les radars ou une combinaison de ces systèmes. Des appartements complets peuvent être équipés de capteurs de mouvement PIP pour « passive infrared sensor » selon une terminologie anglo-saxonne, de capteurs de gazinière, des capteurs dans le lit, des capteurs au sol ..., ce qui permet de donner des schémas d'activités de la vie quotidienne. Cependant, ils ne sont pas capables de donner des informations plus fines sur l'analyse de la marche pour la détection des changements.
Pour les systèmes radar et RGBI-D, des défis ouverts restent à relever afin de déployer et d'utiliser ces systèmes dans des scénarios pratiques à domicile ou dans des établissements spécialisés :

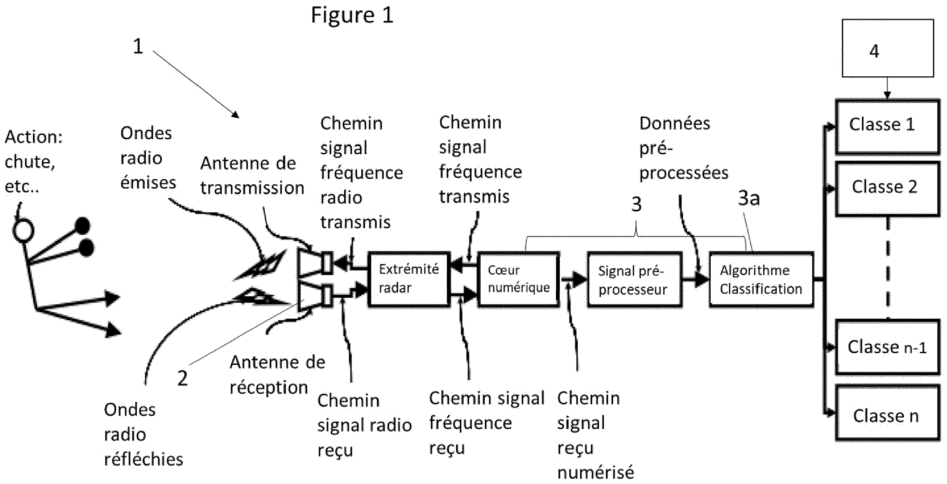

- Concernant les caméras [6], les principaux défis à relever restent les occlusions (zones de pixels mortes), le fonctionnement de nuit, les zones de pixels mortes en 3D, la précision, la résolution des caméras, et le respect de la vie privée. La surveillance des sujets dans leur vie quotidienne pose un réel problème de confidentialité (figure 1). En effet, en fonction du capteur, la perception de l'intrusion et du respect de la vie privée sont différents. Cette perception se traduit par une modification des comportements et l'ajout de
- Concernant les systèmes radar [7], l'environnement intérieur susceptibles de générer des cibles multi-trajet et la réglementation des émissions sont contraignants.

Bien qu'il y ait plus de défis technologiques avec le radar, le fait qu'il n'y ait pas de problème juridique concernant les droits d'image et qu'aucune image de sujet ne soit prise, respectant ainsi la vie privée, facilite l'acceptation des utilisateurs finaux et des investisseurs. Pour les raisons susmentionnées, la modalité radar est une piste de recherche intéressante encore inexploitée en milieu spécialisé (EHPAD), en milieu carcéral ou dans les smart homes.

Le radar est considéré comme une technologie émergente pour la surveillance de la santé et la détection des chutes dans la vie assistée en raison d'un certain nombre d'attributs non partagés par d'autres modalités de détection. La méthode la plus courante pour classifier les activités est basée sur l'extraction de caractéristiques issues des signatures micro-doppler (spectrogramme). Le mouvement relatif des composantes structurelles d'un objet / corps génère des zones de pixels uniques dans le domaine temps-fréquence des retours radar. Par conséquent, différentes activités génèrent des caractéristiques distinctives uniques dans les signatures micro-doppler qui peuvent être utilisées pour la classification. En général, les individus mesurent et extraient différentes caractéristiques des spectrogrammes (temps lent, Doppler), suivies de différentes classifications automatiques. Les techniques pour la classification automatique comprennent l'analyse discriminante de Fisher (FDA), les voisins K-plus proches (KNN), la classification Naïve Bayésienne (NB), et les machines à vecteurs de support (SVM).

Récemment, avec l'augmentation de la puissance de calcul, il est devenu possible d'utiliser des méthodes de « deep learning » selon une terminologie anglo-saxonne. Le « deep learning », ou apprentissage profond, regroupe l'ensemble des méthodes d'apprentissage automatique, supervisées ou non-supervisées, et est capable de déterminer automatiquement les caractéristiques les plus pertinentes à des fins de classification.

Par exemple, pour reconnaître un visage en vision par ordinateur avec les réseaux convolutionnels de neurones (CNN), la première couche peut reconnaître les bords à différents angles, puis dans la seconde couche différentes parties du visage (yeux, bouche, nez), puis superposer des faces entières et essayer ensuite de classifier ce qu'il voit.

Une autre classe d'architectures d'apprentissage en profondeur utilisées pour le traitement de la parole naturelle sont les réseaux neuronaux récurrents (RNN) avec des unités récurrentes bloquées et la mémoire à long terme (LST).

La plupart de ces approches permettent de démontrer que le traitement des signaux issus des spectrogrammes d'un radar rend possible la détection des activités humaines. Les résultats obtenus sont issus de traitement offline sur des bases de données.

Les dimensions embarquées et implémentation temps-réel des traitements ne sont pas abordées. Celles-ci nécessitent de concevoir les algorithmes de classification en prenant en compte dès la conception des contraintes de temps de calcul (débits IO), d'implémentation efficace des traitements en respectant des contraintes supplémentaires de consommation.

## EXPOSE DE L'INVENTION

Pour répondre à ces objectifs, un nouveau système basé sur le développement d'une architecture de radar logiciel émettant à la fréquence (bande entre 6 MHz et 250 GHz, de préférence bande 2 - 4 GHz, de façon plus préférée entre 2,3 et 2,5 GHz) a été développé (figure 1).

Le caractère logiciel du radar rend flexible la forme d'onde qui est émise ainsi que le traitement des signaux, au pied de l'antenne. Ainsi, le traitement des signatures radars de type micro-doppler par des solutions d'algorithmes de traitement d'images et de « machine learning » selon une terminologie anglo-saxonne ou d'apprentissage automatique, permet de caractériser l'actimétrie d'un sujet.

L'invention repose sur l'agrégation de plusieurs techniques en vue de répondre à une problématique. Le caractère inventif comprend deux parties :

- Une technique d'extraction de paramètres de forme géométrique issue de technique de vision par ordinateur, sur des images de type spectrogrammes micro-doppler à hautes résolutions représentant des cartes de vitesse / distance.
- Une technique de classification simple, par exemple statistique , permettant le calcul en temps réel ; par exemple une classification binaire ou multiclasse de type SVM.

L'utilisation conjointe de ces deux techniques, permet d'obtenir des performances supérieures à l'état de l'art (approches « deep ») tout en garantissant une implémentation matérielle plus faible (compatible avec un objet connecté de faible puissance de calcul) et une consommation moindre.

La présente invention présente un dispositif pour caractériser en temps réel l'actimétrie d'un sujet, présentant :

- un radar émettant et recevant des signaux radar, et présentant une interface logicielle pour configurer la forme du signal émis ;
- des moyens de traitement et de calcul couplés au radar, présentant un classifieur entrainé à l'aide d'une base de données.

Lesdits moyens de traitement et de calcul sont configurés pour réaliser en temps réel:

- une acquisition d'images micro-doppler couleurs ayant plusieurs canaux de couleurs (R, V, B), présentant chacune des signatures micro-doppler avec des pixels couleurs dont les valeurs d'intensités se répartissent sur une échelle graduelle;

- un traitement des images micro-doppler pour :

✓ calculer une image dite monochromatique ayant des pixels monochromatiques, ayant chacun une intensité monochromatique donnée, à partir des pixels couleurs de chaque image micro-doppler couleur ;

✓ transformer l'image monochromatique en image binaire par segmentation, selon un seuil d'intensité, des pixels monochromatiques, en réalisant des pixels binaires, dont la valeur binaire pour chaque pixel binaire est fonction de la valeur de l'intensité chromatique du pixel monochromatique associé ou correspondant au pixel binaire (à la même position sur l'image binaire que la position du pixel monochromatique sur l'image chromatique), par rapport à la valeur du seuil, en formant, sur la surface de l'image binaire, des zones segmentées (ou parties délimitées de la surface de l'image binaire) qui présentent des pixels binaires de même valeur binaire, et qui sont issues de la transformation de chaque signature micro-doppler;

✓ calculer des valeurs de paramètres sur chaque zone segmentée, chaque paramètre étant uniquement un paramètre qui caractérise la forme géométrique des zones segmentées ;

✓ classer chaque image binaire dans une classe se rapportant à l'actimétrie du sujet, en fonction des valeurs des paramètres calculés pour toutes les zones segmentées de l'image binaire, à l'aide du classifieur entraîné.

## DESCRIPTION DES FIGURES

D'autres objectifs, caractéristiques et avantages ressortiront de la description détaillée qui suit en référence aux dessins donnés à titre illustratif et non limitatif parmi lesquels :

- la figure 1 représente un schéma de principe de l'invention ;
- la figure 2 représente des signatures micro-doppler qui vont être traitées par le dispositif et procédé selon l'invention ; les signatures micro-dopplers sont les suivantes : (a) Assis sur une chaise ; (b) Levé d'une chaise ; (c) Flexion et récupération d'un stylo à terre ; (d) Flexion et lace ses lacets ; (e) Tomber en avant ; (f ) Accroupissement pour regarder au-dessus et en dessous d'un meuble ;
- la figure 3 représente une chaîne algorithmique de traitement des signatures micro-doppler et qui fait l'objet de la présente invention ;

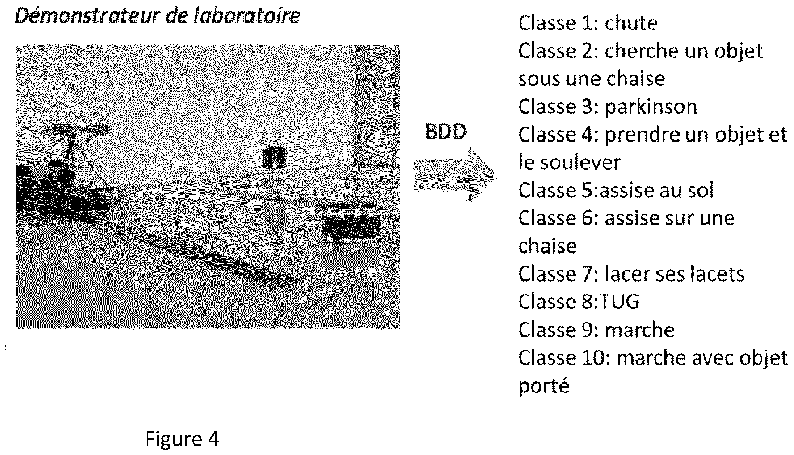

- la figure 4 représente une preuve de concept de laboratoire et les classes considérées;

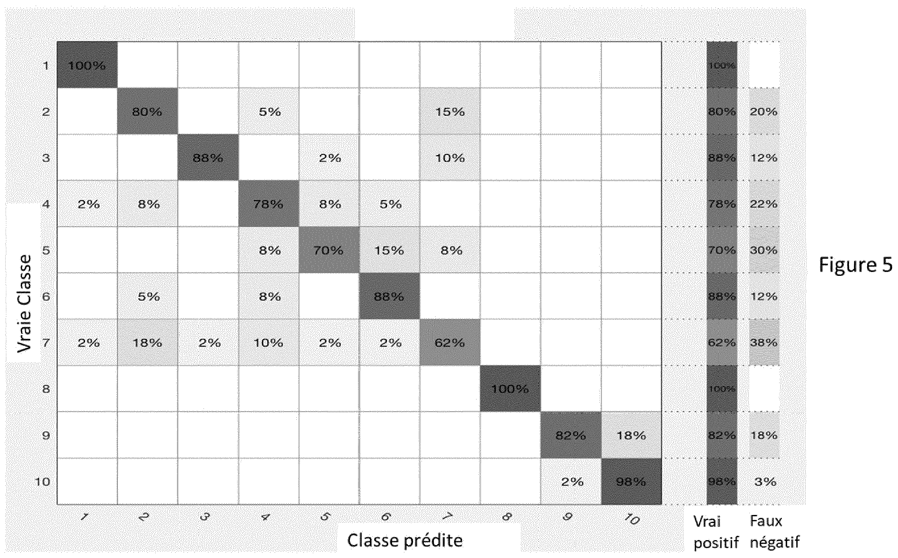

- la figure 5 représente une matrice de confusion.

## DESCRIPTION GENERALE DE L'INVENTION

La présente invention concerne un dispositif 1 pour caractériser en temps réel l'actimétrie d'un sujet, présentant :

- un radar 2 émettant et recevant des signaux radar 2, et présentant une interface logicielle pour configurer la forme du signal émis ;
- des moyens de traitement et de calcul 3 couplés au radar 2, présentant un classifieur 3a entrainé à l'aide d'une base de données.

Lesdits moyens de traitement et de calcul 3 sont configurés pour réaliser en temps réel:

- une acquisition d'images micro-doppler 6 couleurs ayant plusieurs canaux de couleurs (R, V, B), présentant chacune des signatures micro-doppler 6a avec des pixels couleurs dont les valeurs d'intensités se répartissent sur une échelle graduelle;

- un traitement des images micro-doppler 6 pour :

✓ calculer une image dite monochromatique ayant des pixels monochromatiques, ayant chacun une intensité monochromatique donnée, à partir des pixels couleurs de chaque image micro-doppler couleur ;

✓ transformer l'image monochromatique en image binaire par segmentation, selon un seuil d'intensité, des pixels monochromatiques, en réalisant des pixels binaires, dont la valeur binaire pour chaque pixel binaire est fonction de la valeur de l'intensité chromatique du pixel monochromatique associé ou correspondant au pixel binaire (à la même position sur l'image binaire que la position du pixel monochromatique sur l'image chromatique), par rapport à la valeur du seuil, en formant, sur la surface de l'image binaire, des zones segmentées 5a (ou parties délimitées de la surface de l'image binaire) qui présentent des pixels binaires de même valeur binaire, et qui sont issues de la transformation de chaque signature micro-doppler 6a;

✓ calculer des valeurs de paramètres sur chaque zone segmentée 5a, chaque paramètre étant uniquement un paramètre qui caractérise la forme géométrique des zones segmentées 5a ;

✓ classer chaque image binaire 5 dans une classe se rapportant à l'actimétrie du sujet, en fonction des valeurs des paramètres calculés pour toutes les zones segmentées 5a de l'image binaire 5, à l'aide du classifieur 3a entraîné.

Les pixels couleurs des signatures micro-doppler ont une intensité dont la valeur est fonction d'une réflectivité et d'une vitesse du sujet, et se répartit sur une échelle graduelle (au sens de continu).

Ainsi, la conversion de l'image doppler couleur en l'image monochromatique peut consister à convertir, pour chaque pixel de la représentation doppler couleur, le triplet de valeurs représentant les niveaux des couleurs primaires de chaque pixel couleur en une valeur entière qui en est la somme, et représentant une luminosité ou une intensité lumineuse ou luminance associée à un pixel de représentation ainsi produite.

Chaque zone géométrique segmentée présente ainsi des premiers pixels binaires de même valeur et qui se distingue du fond de la surface de l'image binaire.

En effet le fond de la surface de l'image binaire présente lui aussi des seconds pixels binaires de même valeur mais d'une valeur différente de celle des premiers pixels binaires.

Ainsi les signatures micro-doppler se trouvent segmentées en plusieurs zones géométriques segmentées.

Le calcul de similarité pour classer les images peut être basé sur un calcul de distance dans un hyperplan de dimension N ou N est la taille des paramètres.

La présente invention vise à éviter de faire les calculs de l'état de l'art sur les valeurs représentées dans les signatures micro-doppler coûteuses en temps, et ne se porte qu'uniquement sur les caractéristiques de forme géométrique des zones segmentées. La transformation par la binarisation des signatures micro-doppler passe d'une information 3D à une information 2D mais permet de faire des calculs rapides et est efficace pour classer les images.

Le présent dispositif 1 présente des moyens de stockage 4 couplés aux moyens de calcul 3 pour stocker le classifieur 3a, les images micro-doppler 6, les images monochromatiques, les images binaires 5 et le classement obtenu des images binaires 5.

Le traitement des images micro-doppler 6 peut balayer chacune des images binaires en temps réel avec une sous-fenêtre coulissante; et pour chaque position de ladite sous-fenêtre coulissante, il est extrait des paramètres de forme géométrique pour classer chaque sous-image extraite de l'image binaire considérée, dans une classe se rapportant à l'actimétrie du sujet.

Cette sous-fenêtre est utilisée pour la cohérence temporelle des classes (ou activités extraites) et procéder à du suivi (en anglais « tracking »); pour réaliser cette opération de « tracking », il est nécessaire d'adapter l'extraction des paramètres géométriques au cours du temps.

Cette adaptation passe par les modifications des paramètres d'acquisition (tels que le temps de mesure, l'intensité lumineuse, la ou les formes des ondes) en fonction des zones segmentées et des classes attendues, pour modifier les prochaines zones segmentées à venir.

Dans une réalisation, le calcul de l'image monochromatique est réalisé avec la couleur grise qui est fonction de la valeur des canaux de couleur (R, V, B) des pixels couleurs, par exemple en suivant la formule : Gris= 0.299*Rouge+0,587*Vert+0.144*Bleu, pour chaque pixel des images microdopplers couleurs qui a une valeur d'intensité rouge, une valeur d'intensité vert et une valeur d'intensité bleu.

Avantageusement, le dispositif 1 est configuré pour caractériser en continu l'actimétrie de la personne, la base de données étant alimentée et augmentée en continu avec le classement obtenu des images binaires 5 réalisé par le classifieur 3a.

Avantageusement, les moyens de traitement et de calcul 3 sont configurés pour filtrer (supprimer) les pixels de même valeur binaire que les pixels des zones segmentées, mais situés à distance des zones segmentées 5a.

Par exemple, il est utilisé un filtrage morphologique érosion et dilatation pour supprimer ces pixels isolés, qui sont situés à plusieurs pixels de distance de ces zones segmentées et qui visiblement ne font pas partie de ces dernières.

Les valeurs des paramètres de forme géométrique de chaque zone segmentée 5a de pixels binaires de même valeur binaire, ne sont pas des valeurs de fréquence, temps, vitesse, puissance, mesurées sur les signatures micro-doppler, comme c'est le cas dans l'état de l'art.

Avantageusement, le classifieur 3a des moyens de calcul 3, classe les images sans utiliser de réseaux de neurones, tels que des réseaux de neurones convolutionnels (CNN), ou des réseaux de neurones multicouches (DNN), trop couteux en temps.

Avantageusement, le dispositif 1 est embarqué, et le classifieur 3a est choisi notamment dans la liste suivante :

- un boosting algorithme ;
- une cascade adaboost ;
- apprentissage actif ("active learning" en terminologie anglo-saxonne) ;
- un classifieur 3a binaire ou et/ou une cascade de classifieurs binaires ;
- un classifieur multi-classe (exemple SVM (en anglais « Support Vector Machine » à noyau cubique).

Le boosting regroupe un ensemble d'algorithmes tels que : Adaboost, LPBoost, TotalBoost, BrownBoost, xgboost, MadaBoost, LogitBoost. Le Boosting est une méthode séquentielle et chaque échantillon est tiré en fonction des performances de la règle de base sur l'échantillon précédent. Le Boosting et sa mise en oeuvre sont décrits en détail dans Freund & Schapire 1999 (Machine Learning Large. Margin Classification Using the Perceptron Algorithm). Dans le cadre d'un apprentissage actif, un algorithme d'apprentissage est capable d'interroger de manière interactive l'utilisateur (ou une autre source d'informations) pour obtenir les sorties souhaitées à de nouveaux points de données.

Les caractéristiques de forme géométrique de chaque zone segmentée 5a de pixels binaires de même valeur sont choisies notamment parmi la liste suivante: surface, périmètre, centroïde de degré 1, de degré 2, orientation, calcul des moments d'ordre 0, 1 et 2 à n, Carré englobant, Ellipse englobante, etc.

De façon plus précise, par exemple, il peut être considéré :

$$\text{Surface} = Surface = \sum_x \sum_y p_{x,y} \; avec \; p_{x,y} \; appartient \; \{forme \; i\}.$$

$P_{x,y}$ représente la valeur du pixel aux coordonnées x,y dans l'image. La surface comptabilise le nombre de pixel ayant la même valeur, '1', dans l'image. Avantageusement, la détection de contour est obtenue par un filtrage convolutionel par le noyau de Sobel.

- Périmètre = Somme des $p_{x,y}$ appartenant au pourtour de la surface. Le périmètre est obtenu en sommant tous les pixels de la même valeur sur le contour de la forme. Un opérateur de Sobel est utilisé pour définir le contour de la surface. Ainsi, l'équation suivante peut être utilisée :

$$Perimetre = \sum_x \sum_y \; p_x.p_y \; avec \; x,y \in \{bordure \; de \; la \; forme \; i\},$$

- Moment d'ordre 0 :

$$Mo = \sum_x \sum_y p_x^i \cdot p_y^j \; avec \; x,y \in \{forme \; i\} \; et \; i \; et \; j = 0$$

- Centroïde de degré 1 (moment d'ordre 1), correspondant au centre de masse de la forme. Il est de coordonnées $g_x$ et $g_y$, et, calculé à partir des équations suivantes :

$$gx = \frac{1}{N}\Sigma_{i=0}^N p_{x_i} \; \text{et} \; gy = \frac{1}{N}\Sigma_{i=0}^N p_{y_i}$$

avec N le nombre de pixel total dans l'image, $p_{xi}$ l'abscisse du pixel et $p_{yi}$ l'ordonnée du pixel. Il peut aussi être obtenu selon l'équation suivante :

$$M1 = \sum_x \sum_y p_x^i \cdot p_y^j \; avec \; x,y \in \{forme \; i\} \; et \; i \; et \; j = 1,$$

- Centroïde de degré 2, il est calculé par exemple à partir de l'équation suivante :

$$M2 = \sum_x \sum_y p\,_x^{\,i}\,.p_y^{\,j}\ avec\ x,y \in \{forme\ i\}\ et\ i\ et\ j = 2$$

- Orientation : obtenue par la détermination de la droite passant par le moment d'ordre 1 et qui minimise la distance des points de contours à la droite.

Exemples :

- pour la surface : comptage des pixels blancs, moment d'ordre 0 (moyennes selon l'axe x et y) ;
- pour le périmètre : détection de contour (algorithme de Sobel) + comptage des pixels blancs ;
- pour le calcul des moments d'ordre 1 et 2 :

$$Mm,n = \ somme(somme(x^m y^n f(x,y)dxdy))$$

- Carré englobant ;
- Ellipse englobante.

Par exemple des polynômes de Legendre peuvent être utilisés.
Dans une réalisation, la segmentation des pixels est réalisée à l'aide d'un seuil binaire fixe. Dans une autre réalisation, la segmentation des pixels est réalisée à l'aide d'un seuil binaire adaptatif ou variable en fonction de la luminance (exemple : dans la publication Otsu [9]).
Les classes peuvent être choisies parmi la liste suivante :

- marche,
- course
- levée,
- assise,
- classe TUG.

Les classes peuvent être choisies parmi la liste suivante :

- marche avec un objet porté avec 2 mains,
- chute,
- assise sur une chaise,
- lacer ses lacets,
- marche parkinsonienne,
- assise au sol,
- ramasser un objet,
- chercher un objet sous une chaise,
- lever d'une chaise et marche.

Avantageusement, les moyens de calcul 3 sont configurés pour stocker les séquences successives de classe obtenues, et déterminer une carte d'activité du sujet, notamment en utilisant des chaînes de Markov, des arbres de décision binaire ou pas, etc..
Dans une réalisation, le radar 2 est mono-statique (un émetteur et un récepteur).
Dans une autre réalisation, le radar 2 est bi-statique (un émetteur et plusieurs récepteurs).
Le radar 2 émet les signaux radar selon une bande de fréquence comprise entre 6 MHz et 250 GHz, de préférence entre 2 et 4 GHz, de façon plus préférée entre 2,3 et 2,5 GHz
L'interface logicielle radar peut permettre d'émettre des ondes choisies par exemple parmi les catégories suivantes :

◦ Onde continue

◦ Onde continue modulée en fréquence: linéaire et non linéaire

◦ Multiplexage par division de fréquence orthogonale

- Code de phase Newman

- M_ary - Clé par décalage de phase

- M_ary - Modulation d'amplitude en quadrature

- Codage de phase porteuse aléatoire

- Codage de phase de porteuse optimisé avec des algorithmes génétiques

∘ OFDM clairsemé

∘ GFDM

∘ Codage de fréquence Costas

∘ Codes Barker

∘ Codes de phase Chirplike

- Codes P1, P2 et Px

- Code Zadoo-chu

- Codes P3, P4 et Golomb

- Codes de phase basés sur NLFM

- Code Ipatov

- Code Huffman

∘ MFSK

∘ Onde continue modulée à fréquence échelonnée

∘ Train cohérent d'impulsions

∘ Train cohérent d'impulsions modulées

∘ Train cohérent de diverses impulsions modulées

∘ Formes d'onde arbitraires

Le seuil s'adapte par rapport à l'image créée à partir du signal.
Le seuil d'intensité lumineuse est lié à la forme d'onde émise, et est conformé par rapport aux intensités lumineuses des micro- images -doppler couleurs elles-mêmes formées à partir du signal radar émis.
La forme d'onde est configurée par rapport à la taille de la pièce et au besoin en ambiguïté, distance et Doppler.
Le signal peut être configuré pour être plus précis ou moins en distance, suivant les besoins.
Les formes d'onde peuvent être configurées pour que les codes soient orthogonaux pour éviter les interférences entre radars qui peuvent opérer dans le même bâtiment et aussi éviter les interférences d'autres appareils alentours
La présente invention concerne aussi un système de contrôle de l'actimétrie d'un sujet, ledit système comprenant :

- un bâtiment avec des murs ;
- un dispositif 1 selon l'invention, ledit dispositif étant intégré dans un mur dudit bâtiment.

Le dispositif 1 peut être couplé avec un dispositif d'alerte et de contrôle, apte à alerter d'une nécessité d'intervention sur le sujet dont l'actimétrie est suivie, si la succession de classes des images binaires 5, déterminée en temps réel, indique comme carte d'activité un comportement à risque.

Comme mentionné ci-dessus, dans ledit système le dispositif 1 comporte :

- un ou plusieurs radars monostatiques ; et/ ou
- un ou plusieurs radars bistatiques.

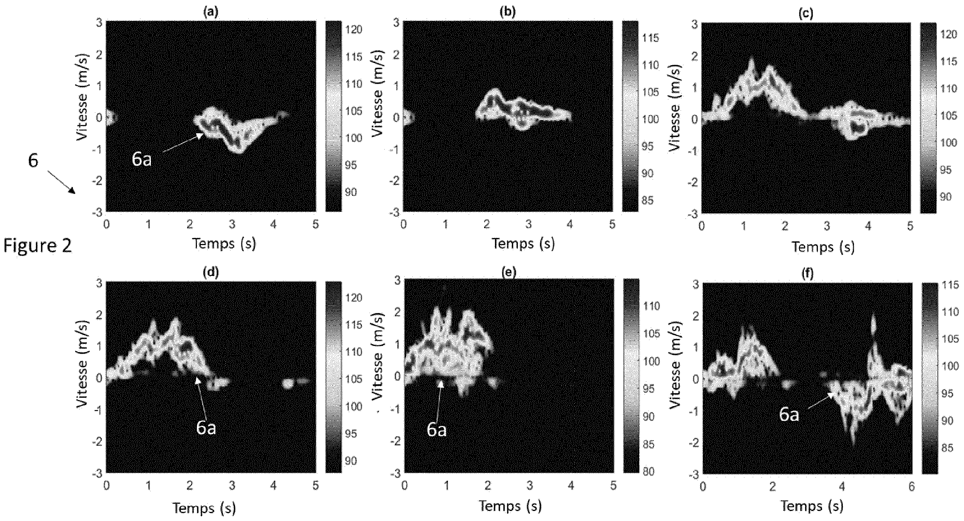

Des premiers traitements ont été développés pour la reconnaissance de l'actimétrie de sujet. Une base de données a été constituée à partir d'un prototype de radar 2 logiciel, composée de 10 classes (marche, marche avec un objet porté avec 2 mains, chute, assise sur une chaise, lace ses lacets, marche parkinsonienne, assise au sol, ramasse un objet, cherche un objet sous une chaise, levée d'une chaise et marche), de 5 sujets. 10 caractéristiques sont extraites des images correspondant à des paramètres de forme géométriques (surface, périmètre, centroïde de degré 1, de degré 2, orientation, etc.). 70% des caractéristiques extraites ont été utilisées pour entraîner des modèles statistiques (SVM - Support Vector Machine à noyau cubique) (figure 2).

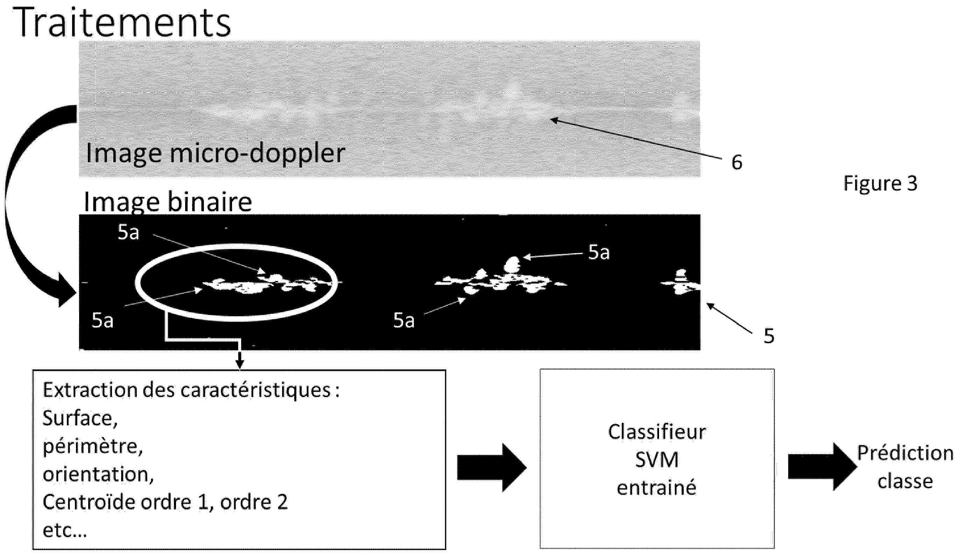

A partir de la base de données, la précision (en anglais « accuracy ») sur l'ensemble des 10 classes est de 84.5% (figure 3). Ces résultats sont comparables à ceux obtenus par des réseaux de neurones convolutionnels - CNN - mais avec une plus faible complexité algorithmique, les rendant plus optimisés pour une implémentation en temps réel.

## APPLICATIONS PRINCIPALES DE L'INVENTION

1. Détection des chutes à domicile, en EHPAD et en milieu hospitalier.

2. Aide à l'évaluation des déficiences de la marche pour les professionnels de la santé (Kinésithérapie par exemple).

La quantification de tests utilisés en routine clinique pour détecter d'éventuelle pathologie ou fragilité. Les trois principaux tests sont le TUG - en anglais « Time Up and Go », le test de l'appui unipodal et le test de Tinetti. Ces tests permettent de fournir une évaluation de la qualité de la marche et de l'équilibre du sujet pour ainsi évaluer son état. A partir des images des spectrogrammes, il est possible de calculer la classe TUG, celle du test de l'appui unipodal ou celle du test de Tinetti.

Ensuite, une fois la classe TUG identifiée par exemple, il devient possible d'extraire des métriques spécifiques et d'objectiver les mesures de TUG, notamment. Les premiers résultats permettent d'identifier un test de TUG avec 100% de précision. Pour cela, des métriques supplémentaires devront être définies pour extraire des qualités métrologiques du TUG.

L'observation répétée de l'activité d'un sujet au cours du temps permettra d'identifier les signes de dégradation de la marche. Après avoir identifié la classe de l'activité (marche, marche lente, ...) des métriques sur la marche peuvent être extraites des spectrogrammes pour caractériser son comportement biomécanique comme la longueur du pas, les vitesses de marche (min, max, moyenne), les asymétries spatio-temporelles, la cadence de marche et l'évolution du centre de gravité. Le suivi de ces paramètres permettra de détecter des variations de comportement et d'identifier des dégradations pouvant amener à des chutes.

3. Détection des suicides en milieu carcérale

Le suivi de l'activité en temps-réel du détenu à risques à partir de la solution ici présenté permettra d'anticiper les situations extrêmes de suicide (risque 7 fois plus élevé que dans le reste de la population) en identifiant des signatures caractéristiques de l'activité.

**Publications** citées :

[1] M. G. Amin, Y. D. Zhang, F. Ahmad, and K. C. D. Ho, "Radar 2 Signal Processing for Elderly Fall Détection: The future for in-home monitoring," IEEE Signal Processing Magazine, vol. 33, no. 2, pp. 71-80, 2016.
[2] (2012). Report to Congress: Aging services Technology Study.
[3] C. Debes, A. Merentitis, S. Sukhanov, M. Niessen, N. Frangiadakis, and A. Bauer, "Monitoring Activities of Daily Living in Smart Homes: Understanding human behavior," IEEE Signal Processing Magazine, vol. 33, no. 2, pp. 81-94, 2016.

[4] H. Li, A. Shrestha, F. Fioranelli, J. L. Kernec, and H. Heidari, "Multisensory Data Fusion for Human Activities Classification and Fall Détection," presented at the IEEE Sensors 2017, Glasgow, UK, 30 Oct - 1 Nov, 2017.

[5] E. Cippitelli, F. Fioranelli, E. Gambi, and S. Spinsante, "Radar 2 and RGB-Depth Sensors for Fall Détection: A Review," IEEE Sensors Journal, vol. 17, no. 12, pp. 3585-3604, 2017.

[6] E. Cippitelli, F. Fioranelli, E. Gambi, and S. Spinsante, "Radar 2 and RGB-Depth Sensors for Fall Détection: A Review," IEEE Sensors Journal, vol. 17, no. 12, pp. 3585-3604, 2017.

[7] M. G. Amin, Y. D. Zhang, F. Ahmad, and K. C. D. Ho, "Radar 2 Signal Processing for Elderly Fall Détection: The future for in-home monitoring," IEEE Signal Processing Magazine, vol. 33, no. 2, pp. 71-80, 2016.

[8] B. Jokanovic and M. Amin, "Fall Détection Using Deep Learning in Range-Doppler Radar 2s," IEEE Transactions on Aerospace and Electronic Systems, vol. PP, no. 99, pp. 1-1, 2017.

[9] Nobuyuki Otsu, « A threshold sélection method from gray-level histograms », IEEE Trans. Sys., Man., Cyber., vol. 9, 1979, p. 62-66

**Revendications**

1. Dispositif (1) pour caractériser en temps réel l'actimétrie d'un sujet, présentant :

    • un radar (2) émettant et recevant des signaux radar et présentant une interface logicielle pour configurer la forme du signal émis ;
    • des moyens de traitement et de calcul (3) couplés au radar (2), présentant un classifieur (3a) entraîné à l'aide d'une base de données,
    lesdits moyens de traitement et de calcul (3) étant configurés pour réaliser en temps réel :

        - une acquisition d'images micro-doppler (6) couleurs ayant plusieurs canaux de couleurs (R, V, B), présentant chacune des signatures micro-doppler (6a) avec des pixels couleurs;
        - un traitement des images micro-doppler (6) pour :

            ✔ calculer une image dite monochromatique ayant des pixels monochromatiques, ayant chacun une intensité monochromatique donnée, à partir des pixels couleurs de chaque image micro-doppler couleur ;
            ✔ transformer l'image monochromatique en image binaire par segmentation, selon un seuil binaire d'intensité lumineuse, des pixels monochromatiques,

                en réalisant des pixels binaires, dont la valeur binaire pour chaque pixel binaire est fonction de la valeur de l'intensité chromatique du pixel monochromatique associé au pixel binaire, par rapport à la valeur du seuil d'intensité,
                en formant, sur la surface de l'image binaire, des zones segmentées (5a) qui présentent des pixels binaires de même valeur binaire, et qui sont issues de la transformation de chaque signature micro-doppler (6a);

            •S calculer des valeurs de paramètres de forme géométrique sur chaque zone segmentée (5a), chaque paramètre de forme géométrique étant uniquement un paramètre qui caractérise la forme géométrique des zones segmentées (5a) ;
            ✔ classer chaque image binaire (5) dans une classe se rapportant à l'actimétrie du sujet, en fonction des valeurs des paramètres calculés pour toutes les zones segmentées (5a) de l'image binaire (5), à l'aide du classifieur (3a) entraîné avec ces paramètres de forme géométriques calculés sur des zones segmentées d'images binaires de sujets tests ayant une actimétrie connue,

    • des moyens de stockage (4) couplés aux moyens de traitement et de calcul (3) pour stocker le classifieur (3a), les images micro-doppler (6), les images monochromatiques, les images binaires (5) et le classement obtenu des images binaires (5).

2. Dispositif (1) selon la revendication 1, dans lequel les paramètres de forme géométrique de chaque zone segmentée (5a) de pixels binaires sont choisis notamment parmi la liste suivante :

    - surface,
    - périmètre,

- centroïde de degré 1,
- centroïde de degré 2,
- orientation,
- calcul des moments d'ordre 0, 1 et 2 à n ,
- carré englobant, et/ou
- ellipse englobante.

**3.** Dispositif (1) selon l'une des revendications précédentes 1 ou 2, dans lequel la segmentation des pixels est réalisée à l'aide d'un seuil binaire d'intensité lumineuse variable en fonction de la luminance.

**4.** Dispositif (1) selon l'une des revendications précédentes, dans lequel la segmentation des pixels est réalisée à l'aide d'un seuil binaire d'intensité luminaire selon la méthode d'Otsu.

**5.** Dispositif (1) selon la revendication 1, dans lequel la segmentation des pixels est réalisée à l'aide d'un seuil binaire d'intensité lumineuse qui est fixe.

**6.** Dispositif (1) selon l'une des revendications précédentes, dans lequel le classifieur (3a) est un classifieur binaire et/ou une cascade de classifieurs binaires.

**7.** Dispositif (1) selon l'une des revendications précédentes, dans lequel les moyens de traitement et de calcul (3) sont configurés pour filtrer les pixels de même valeur binaire que les pixels des zones segmentées, mais situés à distance des zones segmentées (5a).

**8.** Dispositif (1) selon l'une des revendications précédentes, dans lequel le dispositif (1) est embarqué.

**9.** Dispositif (1) selon l'une des revendications précédentes, dans lequel le traitement des images micro-doppler (6) balaye chacune des images binaires (5) en temps réel avec une sous-fenêtre coulissante ; et pour chaque position de ladite sous-fenêtre coulissante, il est extrait des paramètres de forme géométrique pour classer chaque sous-image extraite de l'image binaire considérée, dans une classe se rapportant à l'actimétrie du sujet.

**10.** Dispositif (1) selon la revendication 9, dans lequel le traitement des images micro-doppler (6) permet avec la sous-fenêtre glissante de réaliser du tracking pour modifier les paramètres d'acquisition tels que le temps de mesure, l'intensité lumineuse, la forme des ondes, en fonction des zones segmentées (5a) et des classes attendues, pour modifier les prochaines zones segmentées (5a) à venir.

**11.** Dispositif (1) selon l'une des revendications précédentes, dans lequel le calcul de l'image monochromatique est réalisé en nuance de gris, ladite nuance de gris étant fonction de la valeur des canaux de couleur (R, V, B) des pixels couleurs.

**12.** Dispositif (1) selon l'une des revendications précédentes, dans lequel la classe est choisie parmi la liste suivante :

- marche ;
- course ;
- levée ;
- assise ;
- classe TUG :

    - marche avec un objet porté avec 2 mains ;
    - chute ;
    - assise sur une chaise ;
    - lacer ses lacets ;
    - marche parkinsonienne ;
    - assise au sol ;
    - ramasser un objet ;
    - chercher un objet sous une chaise ;
    - lever d'une chaise et marche.

**13.** Système de contrôle de l'actimétrie d'un sujet, ledit système comprenant :

- un bâtiment avec des murs ;
- un dispositif (1) selon l'une quelconque des revendications 1 à 12, ledit dispositif étant intégré dans un mur dudit bâtiment.

**14.** Système selon la revendication précédente, dans lequel le dispositif (1) est couplé avec un dispositif d'alerte et de contrôle, apte à alerter d'une nécessité d'intervention sur le sujet dont l'actimétrie est suivie, si la succession de classes des images binaires (5), déterminée en temps réel, indique comme carte d'activité un comportement à risque.


**Patentansprüche**

**1.** Vorrichtung (1) zum Charakterisieren der Aktimetrie einer Person in Echtzeit, aufweisend:

• ein Radar (2), das Radarsignale aussendet und empfängt und eine Softwareschnittstelle zum Konfigurieren der Form des ausgesendeten Signals aufweist;
• ein Verarbeitungs- und Berechnungsmittel (3), das mit dem Radar (2) gekoppelt ist und einen Klassifikator (3a), der mithilfe einer Datenbank trainiert wird, aufweist, wobei das Verarbeitungs- und Berechnungsmittel (3) dazu konfiguriert ist, in Echtzeit Folgendes auszuführen:

- eine Aufnahme von Mikro-Doppler-Farbbildern (6) mit mehreren Farbkanälen (R, G, B), von denen jeder Mikro-Doppler-Signaturen (6a) mit Farbpixeln aufweist;
- eine Verarbeitung von Mikro-Doppler-Bildern (6) für:

•S Berechnen eines monochromatischen Bilds mit monochromatischen Pixeln, von denen jedes eine bestimmte monochromatische Intensität hat, auf der Grundlage der Farbpixel jedes Mikro-Doppler-Farbbilds;
✓ Umwandeln des monochromatischen Bilds in ein binäres Bild durch Segmentierung der monochromatischen Pixel gemäß einem binären Lichtintensitätsschwellenwert,

durch Erzeugen binärer Pixel, wobei ein binärer Wert davon für jedes binäre Pixel eine Funktion des Werts der chromatischen Intensität des monochromatischen Pixels ist, das dem binären Pixel zugeordnet ist, in Bezug auf den binären Lichtintensitätsschwellenwert,
durch Bilden segmentierter Bereiche (5a) auf der Oberfläche des binären Bilds, die binäre Pixel mit demselben binären Wert aufweisen und die sich aus der Transformation jeder Mikro-Doppler-Signatur (6a) ergeben;

✓ Berechnen geometrischer Formparameterwerte für jeden segmentierten Bereich (5a), wobei jeder geometrische Formparameter lediglich ein Parameter ist, der eine geometrische Form segmentierter Bereiche (5a) charakterisiert,
✓ Klassifizieren jedes binären Bilds (5) in einer Klasse, die sich auf die Aktimetrie der Person bezieht, in Abhängigkeit von den Werten der Parameter, die für alle segmentierten Bereiche (5a) des binären Bilds (5) berechnet wurden, unter Verwendung des Klassifikators (3a), der mit diesen geometrischen Formparametern trainiert wurde, die auf segmentierten Bereiche der binären Bilder von Testpersonen mit bekannter Aktimetrie berechnet werden,

• ein Speichermittel (4), das mit dem Verarbeitungs- und Berechnungsmittel (3) gekoppelt ist, um den Klassifikator (3a), die Mikro-Doppler-Bilder (6), die monochromatischen Bilder, die binären Bilder (5) und die aus den binären Bildern (5) erhaltene Klassifizierung zu speichern.

**2.** Vorrichtung (1) nach Anspruch 1, wobei die geometrischen Formparameter jedes segmentierten Bereichs (5a) binärer Pixel aus der folgenden Liste ausgewählt werden:

- Oberfläche,
- Umfang,
- Zentroid ersten Grades,
- Zentroid zweiten Grades,

- Orientierung,
- Berechnung der Momente nullter, erster und zweiter bis n-ter Ordnung,
- Begrenzungsquadrat und/oder
- Begrenzungsellipse.

3. Vorrichtung (1) nach einem der vorhergehenden Ansprüche 1 oder 2, wobei die Segmentierung der Pixel unter Verwendung eines binären Schwellenwerts mit variabler Lichtintensität in Abhängigkeit von der Leuchtdichte durchgeführt wird.

4. Vorrichtung (1) nach einem der vorhergehenden Ansprüche, wobei die Segmentierung der Pixel unter Verwendung eines binären Lichtintensitätsschwellenwerts gemäß dem Otsu-Verfahren durchgeführt wird.

5. Vorrichtung (1) nach einem der vorhergehenden Ansprüche, wobei die Segmentierung der Pixel unter Verwendung eines binären Lichtintensitätsschwellenwerts durchgeführt wird, der fest ist.

6. Vorrichtung (1) nach einem der vorhergehenden Ansprüche, wobei der Klassifikator (3a) ein binärer Klassifikator und/oder eine Kaskade binärer Klassifikatoren ist.

7. Vorrichtung (1) nach einem der vorhergehenden Ansprüche, wobei das Verarbeitungs- und Berechnungsmittel (3) dazu konfiguriert ist, die Pixel mit demselben binären Wert wie die Pixel der segmentierten Bereiche zu filtern, die sich jedoch in einem Abstand von den segmentierten Bereichen (5a) befinden.

8. Vorrichtung (1) nach einem der vorhergehenden Ansprüche, wobei die Vorrichtung (1) integriert ist.

9. Vorrichtung (1) nach einem der vorhergehenden Ansprüche, wobei bei der Verarbeitung der Mikro-Doppler-Bilder (6) jedes der binären Bilder (5) in Echtzeit mit einem verschiebbaren Unterfenster abgetastet wird; und für jede Position des verschiebbaren Unterfensters werden geometrische Formparameter extrahiert, um jedes aus dem betrachteten binären Bild extrahierte Unterbild in einer Klasse zu klassifizieren, die sich auf die Aktimetrie der Person bezieht.

10. Vorrichtung (1) nach Anspruch 9, wobei die Verarbeitung der Mikro-Doppler-Bilder (6) es ermöglicht, mit dem verschiebbaren Unterfenster eine Verfolgung durchzuführen, um Aufnahmeparameter wie die Messzeit, die Lichtintensität und die Wellenform in Abhängigkeit von den segmentierten Bereichen (5a) und erwarteten Klassen zu modifizieren, um die nächsten anstehenden segmentierten Bereiche (5a) zu modifizieren.

11. Vorrichtung (1) nach einem der vorhergehenden Ansprüche, wobei die Berechnung des monochromatischen Bilds in Grautönen durchgeführt wird, wobei die Grautöne in Abhängigkeit von dem Wert der Farbkanäle (R, G, B) der Farbpixel sind.

12. Vorrichtung (1) nach einem der vorhergehenden Ansprüche, wobei die Klasse aus der folgenden Liste ausgewählt wird:

- Gehen;
- Laufen;
- Aufstehen;
- Sitzen;
- TUG-Klasse;
- Gehen mit einem Gegenstand, der in beiden Händen getragen wird;
- Fallen;
- Sitzen auf einen Stuhl;
- Schnürsenkel binden;
- Gehen mit Parkinson;
- Sitzen auf dem Boden;
- Aufheben eines Gegenstands;
- Suchen nach einem Gegenstand unter einem Stuhl;
- Aufstehen von einem Stuhl und gehen.

13. System zum Steuern der Aktimetrie einer Person, wobei das System Folgendes umfasst:

- ein Gebäude mit Mauern;
- eine Vorrichtung (1) nach einem der Ansprüche 1 bis 12, wobei die Vorrichtung in eine Wand des Gebäudes eingebaut ist.

**14.** System nach dem vorhergehenden Anspruch, wobei die Vorrichtung (1) mit einer Alarm- und Kontrollvorrichtung gekoppelt ist, die darauf ausgelegt ist, auf die Notwendigkeit eines Eingreifens bei der Person aufmerksam zu machen, dessen Aktimetrie überwacht wird, wenn die Klassenfolge der binären Bilder, die in Echtzeit bestimmt wird, ein risikobezogenes Verhalten als Aktivitätskarte anzeigt.

**Claims**

**1.** A device (1) for **characterising in** real-time the actimetry of a subject, having:

• a radar (2) emitting and receiving radar signals, and having a software interface for configuring a shape of the emitted signal;
• processing and computing means (3) coupled to the radar (2), having a classifier (3a) trained using a database, said processing and computing means (3) being configured to carry out in real-time:

- an acquisition of colour micro-Doppler images (6) having several colour channels (R, G, B) each having micro-Doppler signatures (6a) with colour pixels;
- a processing of the micro-Doppler images (6) to:

✓ compute a so-called monochromatic image having monochromatic pixels, each having a given monochromatic intensity, based on the colour pixels of each colour micro-Doppler image;
✓ transform the monochromatic image into a binary image by segmentation, according to a luminous intensity binary threshold, of the monochromatic pixels,

by creating binary pixels, wherein the binary value for each binary pixel depends on a value of the chromatic intensity of the monochromatic pixel associated with the binary pixel, with respect to the luminous intensity binary threshold,
by forming, over a surface of the binary image, segmented areas (5a) which have binary pixels with the same binary value, and which result from the transformation of each micro-Doppler signature (6a);

✓ compute values of geometrical shape parameters on each segmented area (5a), each geometrical shape parameter being only a parameter that characterises a geometrical shape of the segmented areas (5a),
✓ classify each binary image (5) in a class relating to the actimetry of the subject, according to the values of the parameters computed for all of the segmented areas (5a) of the binary image (5), using the classifier (3a) trained with these geometrical shape parameters computed on segmented areas of binary images of test subjects having a known actimetry,

• storage means (4) coupled to the processing and computing means (3) for storing the classifier (3a), the micro-Doppler images (6), the monochromatic images, the binary images (5) and the classification obtained from the binary images (5).

**2.** The device (1) according to claim 1, wherein the geometrical shape parameters of each segmented area (5a) of binary pixels are selected in particular from among the following list:

- surface,
- perimeter,
- 1st-degree centroid,
- 2nd-degree centroid,
- orientation,
- computation of the 0th-, 1st- and 2nd- to nth-order moments,
- bounding square, and/or
- bounding ellipse.

3. The device (1) according to one of the preceding claims 1 or 2, wherein the segmentation of the pixels is carried out using a luminous intensity binary threshold variable as a function of the luminance.

4. The device (1) according to one of the preceding claims, wherein the segmentation of the pixels is carried out using a luminous intensity binary threshold according to Otsu's method.

5. The device (1) according to claim 1, wherein the segmentation of the pixels is carried out using a luminous intensity binary threshold which is fixed.

6. The device (1) according to one of the preceding claims, wherein the classifier (3a) is a binary classifier and/or a cascade of binary classifiers.

7. The device (1) according to one of the preceding claims, wherein the processing and computing means (3) are configured to filter the pixels with the same binary value as the pixels of the segmented areas, but located at a distance from the segmented areas (5a).

8. The device (1) according to one of the preceding claims, wherein the device (1) is an embedded one.

9. The device (1) according to one of the preceding claims, wherein processing of the micro-Doppler images (6) scans each of the binary images (5) in real-time with a sliding sub-window;
and for each position of said sliding sub-window, geometrical shape parameters are extracted to classify each sub-image extracted from the considered binary image, in a class relating to the actimetry of the subject.

10. The device (1) as claimed in claim 9, wherein processing of the micro-Doppler images (6) allows, with the sliding sub-window, carrying out tracking to modify the acquisition parameters such as the measurement time, the luminous intensity, the shape of the waves, depending on the segmented areas (5a) and the expected classes, to modify the next upcoming segmented areas (5a).

11. The device (1) according to one of the preceding claims, wherein the computation of the monochromatic image is carried out in shades of grey, said shades of grey depending on a value of the colour channels (R, G, B) of the colour pixels.

12. The device (1) according to one of the preceding claims, wherein the class is selected from among the following list:

- walking,
- running
- standing up,
- sitting,
- TUG class,
- walking with an object carried in 2 hands,
- falling,
- sitting on a chair,
- tying shoelaces,
- Parkinsonian gait,
- sitting on the ground,
- picking up an object,
- searching for an object under a chair,
- standing up from a chair and walking.

13. A system for controlling the actimetry of a subject, said system comprising:

- a building with walls;
- a device (1) according to any one of claims 1 to 12, said device being integrated into a wall of said building.

14. The system according to the preceding claim, wherein the device (1) is coupled with an alert and control device, intended to alert on a need for intervention on the subject whose actimetry is monitored, if the sequence of classes of the binary images (5), determined in real-time, indicates a risky behaviour as an activity map.

# Figure 1

Action: chute, etc..

Ondes radio émises

1

Antenne de transmission

Chemin signal fréquence radio transmis

Chemin signal fréquence transmis

Données pré-processées

3    3a

4

Classe 1

Classe 2

Extrémité radar

Cœur numérique

Signal pré-processeur

Algorithme Classification

Ondes radio réfléchies

2

Antenne de réception

Chemin signal radio reçu

Chemin signal fréquence reçu

Chemin signal reçu numérisé

Classe n-1

Classe n

EP 4 042 194 B1

Figure 2

18

# Traitements

Image micro-doppler — 6

Image binaire

5a 5a 5a 5a — 5

Extraction des caractéristiques :
Surface,
périmètre,
orientation,
Centroïde ordre 1, ordre 2
etc...

Classifieur
SVM
entrainé

Prédiction
classe

Figure 3

EP 4 042 194 B1

*Démonstrateur de laboratoire*

Classe 1: chute
Classe 2: cherche un objet sous une chaise
Classe 3: parkinson
Classe 4: prendre un objet et le soulever
Classe 5:assise au sol
Classe 6: assise sur une chaise
Classe 7: lacer ses lacets
Classe 8:TUG
Classe 9: marche
Classe 10: marche avec objet porté

BDD

Figure 4

Figure 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **M. G. AMIN ; Y. D. ZHANG ; F. AHMAD ; K. C. D. HO.** Radar 2 Signal Processing for Elderly Fall Détection: The future for in-home monitoring. *IEEE Signal Processing Magazine,* 2016, vol. 33 (2), 71-80
- *Report to Congress: Aging services Technology Study.,* 2012
- **C. DEBES ; A. MERENTITIS ; S. SUKHANOV ; M. NIESSEN ; N. FRANGIADAKIS ; A. BAUER.** Monitoring Activities of Daily Living in Smart Homes: Understanding human behavior. *IEEE Signal Processing Magazine,* 2016, vol. 33 (2), 81-94
- **H. LI ; A. SHRESTHA ; F. FIORANELLI ; J. L. KERNEC ; H. HEIDARI.** Multisensory Data Fusion for Human Activities Classification and Fall Détection. *IEEE Sensors 2017, Glasgow, UK,* 30 Octobre 2017
- **E. CIPPITELLI ; F. FIORANELLI ; E. GAMBI ; S. SPINSANTE.** Radar 2 and RGB-Depth Sensors for Fall Détection: A Review. *IEEE Sensors Journal,* 2017, vol. 17 (12), 3585-3604
- **B. JOKANOVIC ; M. AMIN.** Fall Détection Using Deep Learning in Range-Doppler Radar 2s. *IEEE Transactions on Aerospace and Electronic Systems,* 2017, vol. PP (99), 1-1
- **NOBUYUKI OTSU.** A threshold sélection method from gray-level histograms. *IEEE Trans. Sys., Man., Cyber.,* 1979, vol. 9, 62-66